# EUROPEAN PATENT APPLICATION

(11) **EP 2 505 074 A2**
(43) Date of publication of application: **03.10.2012**
(21) Application number: 12161581.9
(22) Date of filing: 27.03.2012
(51) Int. Cl.: A23L 1/0522, A23L 1/0526, A23L 1/308

(54) **Use of whole grain - hydrocolloid complexes produced by heat-moisture treatment for satiety, reduction of food intake and weight management**

(30) Priority: 31.03.2011 US 201113077393
(71) Applicant: CORN Products Development Inc., Westchester, IL 60154 (US)
(72) Inventor: Finocchiaro, Eugene Terry, West Amwell, New Jersey 08530 (US); Janik, Danuta Maria, Lawrenceville, New Jersey 08648 (US)
(74) Representative: Held, Stephan

(57) **Abstract**

The present invention relates to whole grain - hydrocolloid complexes, their preparation and their use in foods. The complexes positively impact the foods into which they are incorporated to give longer-lasting and/or more potent satiety, preferably without adversely affecting texture and processing thereby enabling the development of commercially viable ingredients for energy management. The invention further relates to the reduction of food intake and/or management of weight by increasing such satiety.

## Description

### Field of the Invention

The present invention relates to whole grain - hydrocolloid complexes produced by heat-moisture treatment and their use in foods.

### Summary of the Invention

The present invention relates to whole grain- hydrocolloid complexes, their preparation and their use in foods. The complexes positively impact the foods into which they are incorporated to give longer-lasting and/or more potent satiety, thereby helping energy management. The invention further relates to the reduction of food intake and/or management of weight by increasing such satiety.

The present invention addresses the major limitations associated with existing state-of-art ingredients for satiety: robust clinical efficacy in a form that is edible and process compatible in food products. The unique combination of whole grain and hydrocolloid enables a more potent or more robust satiety effect by combining two physiological satiety mechanisms. Further, the unique complexation of these two materials enables higher whole grain content and improved texture by controlling the hydration of hydrocolloid, particularly in the food form, without adversely affecting clinical efficacy. Typically, when high levels of commercially available whole grain and hydrocolloid are incorporated into foods, processability and/or eating quality suffers. This invention allows for higher levels of whole grain while minimizing the deleterious effects of hydrocolloids in foods, thus enabling superior eating quality and textural benefits vs. a comparable "dry blend" control which may have higher water-binding and gumminess from the uncomplexed hydrocolloid.

The term "complex" is meant to include two or more ingredients that have been co-processed to form a material in which the ingredients are not physically separable.

The term "dry blend" is meant to include two or more ingredients combined to form a material in which the ingredients are physically separable.

The term "hydrocolloid" is meant to include any viscosifying gum with a neutral charge (non-ionic).

The term "total dietary fiber content" (TDF) is measured by weight of undigested material separated by filtration as described by the test described as AOAC Method 991.43. In one embodiment, this may include the polysaccharides and remnants of plant materials that are resistant to hydrolysis (digestion) by human alimentary enzymes, including non-starch polysaccharides, resistant starch, lignin and minor components such as waxes, cutin, and suberin.

The term "resistant starch (RS)" is defined as the sum of starch and starch degradation products that are not absorbed in the small intestine of healthy individuals and is measured by treatment with pancreatic alpha-amylase and amyloglucosidase (AMG) using a modification of the Englyst method, described in the Examples section. It is inclusive of all resistant starch known in the art. Resistant starch product is intended to mean a product containing resistant starch.

"High resistant starch content" is intended to mean a resistant starch content of at least 70% by weight based on the weight of the starch.

The term "whole grain", as used herein, is intended to not only include the cereal grain itself, but also is intended to include those which have been partially processed by methods well known in the art including, for example, dry milled grains such as grits, meals, kernels and flour. It is not intended to include cereal grains which have been processed to remove part of the grain; it is not intended to include starch.

The term "high amylose" is used herein, is defined as containing at least 27% amylose for wheat or rice and at least about 50% amylose for other sources, particularly at least about 70%, more particularly at least about 80% amylose by weight based of the starch. The percent amylose is determined by using the potentiometric test described in the Examples section.

The term "increased satiety", as used herein, is intended to mean that the caloric intake at least within the two hours after consumption of the complex is reduced by a statistically significant amount compared to consumption of a readily digestible 10 DE (dextrose equivalent) maltodextrin of equal caloric content (e.g., STAR-DRI 100, commercially available from Tate & Lyle, Decatur, Illinois, USA).

The term "mammal", as used herein, is intended to include humans.

### Detailed Description of the Invention

The present invention relates to whole grain-hydrocolloid complexes, their preparation and their use in foods to increase satiety in mammals. The invention also relates to the reduction of caloric intake as a consequence of inducing satiety, which will aid in weight management.

The whole grain component of the complex may be derived from any native source. A native source, as used herein, is one as it is found in nature. Also suitable are grains derived from a plant obtained by standard breeding techniques including crossbreeding, translocation, inversion, transformation or any other method of gene or chromosome engineering to include variations thereof. In addition, whole grain derived from a plant grown from induced mutations and variations of the above generic composition which may be produced by known standard methods of mutation breeding are also suitable herein.

Typical sources for the whole grain are cereals and includes without limitation corn (maize), barley, wheat, rice, rye, oats, amaranth, arrowroot, canna, or sorghum, as well high amylose varieties thereof. In one embodiment, the source for the whole grain is a high amylose grain.

In another embodiment, the plant source is one having an amylose extender genotype, the starch of the whole grain comprising less than 10% by weight amylopectin. This grain is derived from a plant breeding population, particularly corn, which is a genetic composite of germplasm selections and its starch comprises at least 75% by weight amylose, optionally at least 85% amylose (i.e., normal amylose) as measured by butanol fractionation/exclusion chromatography techniques. The starch further comprises less than 10%, by weight, optionally less than 5%, amylopectin and additionally from about 8 to 25% low molecular weight amylose. The grain is preferably derived from a plant having a recessive amylose extender genotype coupled with numerous amylose extender modifier genes. This grain and its method of preparation are described in U.S. Pat. No. 5,300,145, the specification of which is incorporated herein by reference.

The whole grain component of the complex may be derived directly from a native source, and/or may be physically, chemically or enzymatically modified. In one embodiment, the whole grain component has high resistant starch content. In another embodiment, the whole grain component is sourced from high amylose corn.

The hydrocolloid component of the complex may be any viscosifying gum with a neutral charge (non-ionic) and is intended to include without limitation guar gum, konjac, locust bean gum, tara gum, and other such exocellular polysaccharides. In one embodiment, the hydrocolloid component is guar gum. In yet another embodiment, the gum is a high viscosity gum with a viscosity specification between 4,000 - 5,500 cPs (1% aqueous solution @ 25 °C using a Brookfield RVT, spindle #4 @ 20 RPM).

The complex has a ratio (wt/wt) of whole grain:hydrocolloid of at least 90:10. In one embodiment, the ratio (wt/wt) of whole grain:hydrocolloid is at least 80:20. In yet another embodiment, the complex has a ratio (wt/wt) of starch:hydrocolloid of no more than 95:5.

In one aspect of the invention, the whole grain is a high amylose corn flour and the hydrocolloid is a guar gum.

The two materials are blended and the blend is subjected to heat-moisture treatment (HMT) to form a complex. Heat-moisture treatment of a single ingredient is well known in the art as exemplified by U.S. Publication No. 2006-0263503 and U.S. Publication No. 2002-0197373, the specifications of which are incorporated herein by reference.

In preparing the complex of this invention, it is necessary that the whole grain - hydrocolloid blend be processed by heat-moisture treatment for a specified time at a specified total water content and defined temperature combination so as to avoid partially or fully gelatinizing the starch of the whole grain so that the starch retains its granular structure. By treating the whole grain under these conditions, a complex will be prepared.

The total water (moisture) content of the blend prior to heat-moisture treatment will be in a range of from about 10 to 50%, and in one embodiment in the range of from about 20 to 30% by weight based on the weight of the dry blend. That is, the moisture content is the water content present in the components plus the added water (if any). In one embodiment, this relative level of moisture is maintained substantially constant throughout the heating step. In another embodiment, no water is added to the blend during heating (i.e., no water is present during the heating step other than the equilibrium moisture content of the components). In another embodiment, the moisture content is not controlled (kept substantially constant) during the heat-moisture treatment such that the resultant complex has a lower moisture content than the blend.

The whole grain - hydrocolloid blend is heat-moisture treated at a target temperature of from about 60 to 160°C, and in one embodiment at a temperature of from about 80 to 120°C. While the most desirable temperature and water content may vary depending on the particular whole grain and hydrocolloid used (including without limitation the source and amount of protein, starch, lipid, and hydrocolloid as well as the particle size of the components), it is important that the starch present in the whole grain remain in the granular state. Granular state is intended to mean that the starch does not lose its crystalline and birefringent characteristics.

The time of heating at the target temperature may vary depending on the whole grain used, its amylose content and particle size, and the hydrocolloid used, as well as the amount of moisture and the heating temperature. In one embodiment, such heating time will be from about 1 minute to 24 hours. In another embodiment, the heat time at the target temperature will be from about 15 minutes to 2 hours.

The heat-up (ramp) time may vary depending upon the equipment used, the process conditions, and the whole grain and hydrocolloid components used. In one embodiment, it is desirable to have a short heat-up time to avoid color and adverse flavor formation in the resultant complex. In another embodiment, the heat-up time is less than about 5 minutes and in another less than about 1 minute.

The conditions for heat-moisture treating the blend to obtain a complex are such that the granular structure of the starch is not destroyed (gelatinized); that is, the starch granules remain crystalline and birefringent. Further, there would be no loss of any Maltese cross present in the native starch of the whole grain component when the granular structure is viewed under polarized light. Under some conditions, such as at high moisture and high temperature, the starch granule may be partially swollen, but the crystallinity is not completely destroyed. Under these conditions, the starch granule has not been destroyed.

The heat-moisture treatment may be conducted in any equipment known in the art, which provides sufficient capabilities for powder processing. In one embodiment the equipment additionally provides sufficient capabilities for one or more of the following: moisture addition, moisture control, mixing, heating and/or drying. In one embodiment, the equipment is a continuous tubular thin film dryer, such as that commercially available from Hosokawa-Bepex (Solidaire dryer). In another embodiment, the equipment is a combination of a continuous thin film dryer in series with a continuous heated conveyer screw, which may optionally be pressurized to control moisture content at the target temperature. In yet another embodiment, the equipment is a batch ploughshare mixer. The heat-moisture treatment may be done as a batch or as a continuous process.

The whole grain - hydrocolloid blend or complex may additionally be processed either before or after the heat-moisture treatment (HMT), as long as such process does not destroy the granular structure of the starch. In one embodiment, such additional processing may include degradation using alpha-amylase or acid treatment and in another embodiment, chemical modification.

The particle size of the whole grain component may be adjusted, before heat-moisture treatment, for example by grinding, agglomerating, and/or sieving. The particle size of the complex may also be adjusted after heat-moisture treatment; however, it should be noted that grinding may reduce the total dietary fiber and/or RS content of the complex.

The components or the complex may be purified using any techniques known in the art. In one embodiment, the whole grain is bleached using methods known in the art to reduce color. The pH of the whole grain or the complex may also be adjusted using methods known in the art. In one embodiment, the pH of the complex is adjusted to between 5.5 and 8.0.

The complex may be dried using any drying means known in the art which will not gelatinize its starch. Drying includes any method known in the art, including without limitation spray drying, flash drying, air drying, freeze drying, vacuum drying, belt drying, and drum drying. In one embodiment, the complex is air dried and in another it is flash dried.

The pre- and/or post- processing methods used may further control the physical or chemical properties of the complex or otherwise make the complex more desirable for use in foods.

In one aspect of the invention, the complex contains no caloric components other than the whole grain and the hydrocolloid.

The resulting complex will contain whole grain, the starch of which has retained its granular structure as evidenced by its birefringent characteristic when viewed under the microscope and by no loss of any Maltese cross present in the native starch when viewed under polarized light.

In one embodiment, the complex will have a total dietary fiber content of at least about 45% (w/w). In another embodiment, the complex will have a total dietary fiber content of at least about 50% (w/w) and in another embodiment at least about 55% (w/w). In one aspect of the invention, the complex will have an absolute increase in total dietary fiber of at least 3% (w/w) higher than that of the dry blend. In yet another aspect, the complex will have an absolute increase in total dietary fiber of at least 4.5% (w/w) higher than that of the dry blend. The level of dietary fiber will vary depending on the conditions used for heat-moisture treatment as well as the particular starting components. The total dietary fiber content is measured by the procedure used in the Examples section.

In one embodiment, the complex will have a resistant starch content of at least about 70% by weight of the starch. In another embodiment, the complex will have a resistant starch content of at least about 75% by weight of the starch, in yet another embodiment at least 80% by weight of the starch, and in still another embodiment at least about 85% by weight of the starch. The level of resistant starch will vary depending on the conditions used for heat-moisture treatment as well as the particular starting components. The resistant starch content is measured by the procedure used in the Examples section.

The resultant complex has high *in vitro* stomach viscosity content. In one embodiment of the invention, the complex has an in vitro stomach viscosity content of at least 90% of area under the curve (AUC) vs. pure, fully hydrated guar gum. In another embodiment of the invention, the complex has an in vitro stomach viscosity content of at least 95% of AUC vs. guar gum. In yet another embodiment of the invention, the complex has an in vitro stomach viscosity content of at least 100% of AUC vs. guar gum. This *in vitro* stomach viscosity content is measured using the method set forth in the Example section.

The complex is fed to a mammal. In one embodiment, the mammal is a companion animal, including without limitation, dogs and cats. In another embodiment, the mammal is a human.

The complex is effective such that consumption is effective to increase satiety by reducing caloric intake at least within the two hours following consumption by at least a statistically significant amount when compared to consumption of a readily digestible 10 DE (dextrose equivalent) maltodextrin of equal caloric content. In one aspect of the invention, this statistically significant amount of caloric intake reduction is at least 10%. In another embodiment, the complex is effective in an amount of at least 7.5 grams, in another embodiment at least 10 grams, in yet another embodiment at least 15 grams, and in still yet another embodiment at least 20 grams. In one aspect, the caloric intake is reduced by at least 15% using any of the above criteria. In a further aspect, the caloric intake is reduced by at least 20% using any of the above criteria. In another aspect, the caloric increase is reduced within the 24 hour period following consumption using the same criteria. Such decreased caloric intake may further result in increased weight loss.

The resultant complex of this invention may be eaten as is or incorporated into a variety of foods that include, but are not limited to, cold form snack bars, baked goods such as muffins and cookies, ready-to-eat cereals, pasta and other low-moisture food systems. Food products are also intended to include nutritional products, including but not limited to, prebiotic and synbiotic compositions, diabetic foods and supplements, dietetic foods, foods to control glycemic response and tablets and other pharmaceutical dosage forms. Food products comprise the complex and at least one additional edible ingredient.

When added to a food product, the resultant complex is added in any amount desired. In one aspect, the complex is added in an amount of from 5 to 75% (w/w) of the food product and in another aspect in an amount of from 10 to 65% (w/w) of the food product. In one embodiment, the complex is added in an amount of at least 10% (w/w) based upon the food. In another embodiment, the complex is added in an amount of at least 15% (w/w) based upon the food. In yet another embodiment, the complex is added in an amount of at least 20% (w/w) based upon the food. In still yet another embodiment, the complex is added in an amount of at least 25% (w/w) based upon the food. In a further embodiment, the complex is added in an amount of at least 30% (w/w) based upon the food. In yet a further embodiment, the complex is added in an amount of at least 35% (w/w) based upon the food. In still yet a further embodiment of the invention, the complex is substituted for at least part of the flour or other carbohydrate-based product conventionally added to the food, for example, by replacing the conventional starch, flour, grits or grain.

Addition of the whole grain-hydrocolloid complex to foods may not significantly affect the organoleptic quality attributes of the food in any deleterious way, including texture (gumminess) or flavor, and may, in some cases, provide favorable organoleptic changes. The addition of the complex to foods may increase the nutritional value of the food, such as the resistant starch and/or dietary fiber content.

### EXAMPLES AND METHODOLOGIES

The following examples are presented to further illustrate and explain the present invention and should not be taken as limiting in any regard. All parts, ratios, and percentages are given by weight and all temperatures in degrees Celsius (°C) unless otherwise noted.

The following ingredients were used throughout the examples.

Hi-maize^{®} Whole Grain Corn Flour, a high amylose corn whole grain flour commercially available from National Starch LLC containing approximately 60% RS and 30% TDF.

Coyote Brand™ Guar Gum HV consisting chiefly of high molecular weight galactomannan commercially available from Gum Technology Corporation.

The following test procedures were used throughout the examples:

### A. Resistant Starch ("RS") Determination (Modified Englyst Method)

Resistant starch content was determined using a modified version of the Englyst Digestion Method (Englyst et. al., European Journal of Clinical Nutrition, vol. 46 (Suppl. 2), pp S33-S50, 1992). The procedure and modifications are detailed below. Rapidly digestible starch (RDS) is defined as the amount of glucose released at 20 minutes; slowly digestible starch (SDS) is defined as the amount of glucose released between 20 minutes and 120 minutes; resistant starch (RS) is the starch not hydrolyzed after 120 minutes of incubation. RS content is determined indirectly by measuring the amount of digested carbohydrate (i.e., free glucose) after 120 min. of incubation, then calculating RS by subtracting the amount of free glucose from carbohydrate to give % RS based on the carbohydrate content.

In vitro glucose release results were adjusted to take into account only the total starch portion of the material and not the total carbohydrate content. The hydrocolloid, lipid and protein amounts were subtracted out of the sample weight assayed based on their percentage in the complex. This was done to determine the RS content based on the total starch portion of the complex. Including the weight of the non-starch materials would result in artificially higher complex RS content.

### Preparation of standard solutions, enzyme solutions, blanks and glucose controls:

a. Reaction "blank" consisted of 20 ml of 0.25M sodium acetate.
b. Glucose standard consisted of 20 ml 0.25M sodium acetate buffer plus 500 mg glucose.
c. Stock solution A was prepared by dissolving 0.5% (w/v) pepsin (porcine stomach mucosa (P7000) from Sigma) plus 0.5% (w/v) guar gum (G-4129 guar gum from Sigma) in 0.05M HCl.
d. Preparation of purified enzyme solution: 12g of porcine pancreatin (Sigma) was dissolved in 85 mls of de-ionized room temperature water. The solution was subsequently centrifuged (3000 rpm, 10 min, 50 ml tubes) and the supernatant was decanted off and saved.
e. Stock solution B was prepared by adding 40 mg of dry invertase (Sigma) and 1.0 ml amyloglucosidase (AMG) (300L AMG from Novozymes) to the aforementioned supernatant.

### Determination of RS Content (Modified Englyst Protocol):

Each test sample was weighed (to the nearest 0.1 mg) to deliver 550 - 600 mg of carbohydrate in each test tube. 10 mls of Solution A was then added to each tube. Samples were covered tightly, mixed, and then incubated in a quiescent water bath @ 37 °C for 30 minutes. Ten mls of 0.25M sodium acetate buffer was added to neutralize the solution. Next, 5 mls of enzyme mixture (solution B) was added to the samples, blank, and glucose tubes @ 20 - 30 second intervals, and placed into the 37 °C water bath for digestion. Tubes were shaken horizontally during digestion. At 120 minutes of digestion time, 0.5 ml aliquots were removed and placed into separate tubes of 19 mls of 66% ethanol to stop the reaction (enzyme will precipitate; re-disperse before next step). 1.0 ml aliquot of the ethanolic solution was then pipetted into 1 ml micro-centrifuge tubes and centrifuged for 5 min. @ 3000 g. Glucose concentrations were subsequently measured using the glucose oxidase/peroxidase (GOPOD) method (Megazyme Kit K-Gluc). Three ml of GOPOD was placed into each culture tube and 0.1 ml of each sample was added, mixed well and incubated for 20 minutes at 50 °C. Free glucose was determined spectrophotometrically for absorbance at 510nm wavelength. The percent glucose (digestion) for each sample is calculated based on the UV absorbance relative to the standards. Routine controls were run that included a reference sample of regular dent corn. All analyses were run at least in duplicates.

### B. Total Dietary Fiber Determination ("TDF") using AOAC Method 991.43:

Total dietary fiber (TDF) was determined using the Megazyme-K-TDFR. diagnostic kit recommended for AOAC Official Method 991.43. Duplicate samples (1.0g dry basis) were dispersed in 0.05M MES/TRIS buffer solution (40ml, pH 8.2) in 400 ml tall-form beaker and a heat stable alpha-amylase solution (50µl) was added. The mixture was incubated in the shaking water bath at 98 °C for 35 minutes. After cooling to 60 °C, the mixture was treated with protease enzyme (100µl) and incubated for 30 minutes. The digest was adjusted to pH 4.5 with HCl solution. Then glucoamylase (200µl) was added and the mixture was digested for another 30 minutes at 60 °C. An insoluble residue was precipitated by adding 4 volumes of 95% ethanol. The residue was collected on a packed filter, dried overnight at 105 °C, weighed and calculated as total dietary fiber (minus the protein and ash contents in residue). All TDF data was reported on a dry basis.

### C. Moisture Content ("%M") Determination:

The moisture content of complexes and bars was determined using a Sartorius electronic moisture analyzer (model MA 30) available from Sartorius AG. Moisture balance was set to 105 °C on "Auto" mode. In this mode, MA 30 recognizes when a considerable weight change is no longer expected (when moisture loss per unit of time reaches zero, or the readout remains constant for a short time after a slight decrease in weight) and automatically ends the moisture determination routine.

### D. Amylose Content by Potentiometric Titration:

0.5g of a starch (1.0g of a ground grain) sample was heated in 10mls of concentrated calcium chloride (about 30% by weight) to 95 °C for 30 minutes. The sample was cooled to room temperature, diluted with 5mls of a 2.5% uranyl acetate solution, mixed well, and centrifuged for 5 minutes at 2000 rpm. The sample was then filtered to give a clear solution. The starch concentration was determined polarimetrically using a 1cm polarimetric cell. An aliquot of the sample (normally 5mls) was then directly titrated with a standardized 0.01N iodine solution while recording potential using a platinum electrode with a KCl reference electrode. The amount of iodine needed to reach the inflection point was measured directly as bound iodine. The amount of amylose was calculated by assuming 1.0 gram of amylose will bind with 200 milligrams of iodine.

### E. Measurement of Guar Gum Viscosity:

The Brookfield viscosity of guar is determined using the procedure listed below (Cold Brookfield Viscosity Analysis Method: B-V-1.03B, Polypro International, Inc.). A sample is dispersed in water and allowed to hydrate; the Brookfield viscosity is read at specified times.

### APPARATUS:

1. Waring blender, consumer model (minimum 360 Watt motor)
2. Quart blender cup (stainless or glass)
3. Variac, 0-140 volts
4. Balance, accurate +/- 0.01 grams
5. Graduated cylinder, 500 ml
6. Beaker, Griffin Low Form, 600 ml
7. Stopwatch
8. Brookfield RV viscometer with spindles
9. Constant temperature water bath
10. Stirring rod
11. Weigh boat
12. Thermometer

### CHEMICALS AND REAGENTS:

Distilled or deionized water (pH adjusted to 5.5-6.0)

### PROCEDURE:

A. Preparation of water
   1. Adjust pH to 5.5-6.0 (use dilute Nitrogen Gas or HCl)
   2. Adjust temperature to 25 °C
B. Calibration
   1. Assure that the Brookfield pointer moves freely and is properly calibrated according to the manufacturer's instructions
   2. Set pH meter to read 7.00 +/- 0.01 with 7.00 buffer
C. Analytical Procedure
   1. Weigh 5.00 +/- 0.01 grams of gum to be tested into a weigh boat
   2. Measure 495 +/- 2 ml of distilled or deionized water into a Waring blender cup set on a blender base
   3. Adjust the speed of the blender so as to form a vortex half way between the blender blade and top of the water (approximately 1500-1800 rpm)
   4. Guar lumps will not go into solution and contribute to inaccurate viscosity readings. Avoid letting the powder come into contact with the walls of the blender cup or hub of mixing blade. To form a lump free solution, direct the gum to the top of the slope of the vortex. Simultaneously start the stopwatch and rapidly dump the guar into the agitating water.
   5. Continue mixing for 2 minutes. As the solution thickens, slightly increase the blade speed to maintain a slight vortex. Keep air entrapment to a minimum. At 2 minutes, transfer solution into a 600 ml Griffin Low Form beaker. Void test if lumps are visible.
   6. Maintain the solution temperature at 25 °C in the constant temperature water bath
   7. Use 20 rpm viscometer speed. Normally, the #3 spindle will be used. The #2 spindle may be required if the viscosity is initially lower than 1300 cps.
   8. Viscosity readings will be taken at 15 minutes, 30 minutes, 1 hour, 2 hours, 4 hours and 24 hours after mix. At 1 minute before the required reading, remove the beaker containing the solution from the bath, stir the solution with a glass rod, place under the viscometer and install the spindle. At 20 seconds before the reading, turn on the viscometer, read at the specified time.
   9. At 2 hours read and record pH. Some time may be required to reach a stable reading due to viscous nature of the solution.

### F. Measurement of Stomach/Intestinal Viscosity:

A bench-top stomach model was developed and includes features derived from other stomach models referenced in the literature (Kimura et al., 2000, National Enzyme Co. / TNO Nutrition and Food Research, 2004). This digestive model also simulates the buffering capacity of stomach components in the "fed" state, as distinguished from other stomach models run in a "fasted" or empty-stomach state. In order to standardize the digestion process and to improve the reproducibility of the procedure in the laboratory, some simplifying assumptions were made:
- Size of the stomach, 1.25 liters.
- Simulation of mastication by stomacher pre-treatment, no salivary amylase used.
- Agitation: By reciprocating shaker platform, 120 rpm, constant throughout run.
- Temperature: 37 °C fixed throughout (vessels immersed in temperature-regulated water bath); normal human body temperature.
- Stomach media components:
   o Pepsin (from porcine gastric mucosa - e.g., Sigma P7000); activated from pepsinogen by low pH conditions, breaks down protein components to peptides and is most active between pH 2-4.
   o Mucin (protective protein; e.g. Type II, Sigma M2378); since pepsin comes from the cells of the stomach walls, it was decided to include the mucin protective proteins in the systems which also derives from the stomach walls.
   ○ pH 5.0 buffer system: potassium hydrogen phthalate (e.g., Sigma 179922)/NaOH (e.g. 1.0N solution, Sigma S2567). Simulates the presence of some food, not as strong a buffer as acetate.
- Stomach stage: 2 hours with HCl addition. Rate of acid HCl addition in stomach stage assumed to be 36 milliequivalents (mEq) per hour at a constant rate (0.50 ml/min of 1.5N HCl for 1.25 liter stomach volume). Acid added drop-wise onto the liquid surface through a metered peristaltic pump. pH shifted from ~5.0 - ~2.0 in 2 hours stomach stage.
- Neutralization: Performed by the addition of 15 ml 6N NaOH (solution from pellets, e.g., Sigma S8045) and 15 g NaHCO₃ (e.g., Sigma S6014): By using a combination of NaOH and NaHCO₃, the pH shift may be accomplished quickly without excess foaming (as in the case of NaHCO₃ alone) and without driving the pH too high above the target pH of 7.0 (as in the case of NaOH alone).
- Intestine stage 2 hours: Enzyme present in intestine: pancreatin (porcine source, e.g., Sigma P8096), a blend of amylase, lipase, protease and ribonuclease. Not included in this simulation are bile acids (also called bile salts).
- Note also that glucose release (GR), as well as resistant starch levels are measured in a separate analytical test (Englyst *et al.,* 1992).

### Master diagram for stomach/intestine in vitro model operation

N. S. STOMACH/UPPER INTESTINE MODEL, Continuous HCl Addition in Stomach w/Neutral Upper Intestine 8/25/2008

| Stage: | 2-liter | Glass reaction flask, 1.25-liter working volume, immersed in 37 deg C water bath | Amount (ml) |
|---|---|---|---|
| | | Prepared active solution (per preparation protocol) | Active + 600g water |
| | 1) | Water | 277.500 |
| | 2) | 0.2 M potassium hydrogen phthalate (40.844 g potassium hydrogen phthalate (Sigma 179922) in 1 liter stock solution) | 207.500 |
| Stomach t=0 | 3) | 1.0 M NaOH (Sigma S2567) | 18.750 |
| | 4) | Gastric mucosa mucin solution (prepare fresh for each batch) (1.5 g gastric mucosa mucin (Sigma M2378) in 50 ml water) | 43.125 |
| | 5) | 0.2% pepsin solution, added at time=0 (prepare fresh for each batch and kept refrigerated prior to usage) (0.20 g pepsin 2500-3500 units/mg (Sigma P7000) in 104 ml stock solution) | 43.125 |
| Stomach t=0 120 | 6) | 1.50 N HCl, start addition at time=0, 0.50 ml/min for 120 min | 60.000 |
| Neutralization | 7) | 6N NaOH solution (24g of NaOH pellets in 100ml water) and NaHCO₃ 15g | 15.000 |
| | | Total | 1250.000 |

| | | | |
|---|---|---|---|
| 15 ml of 6 N NaOH (which gives 90 mmol OH- to match the 90 mmol of H+), then 15 g of sodium bicarbonate (which doesn't foam since the Elapsed Time | | | |

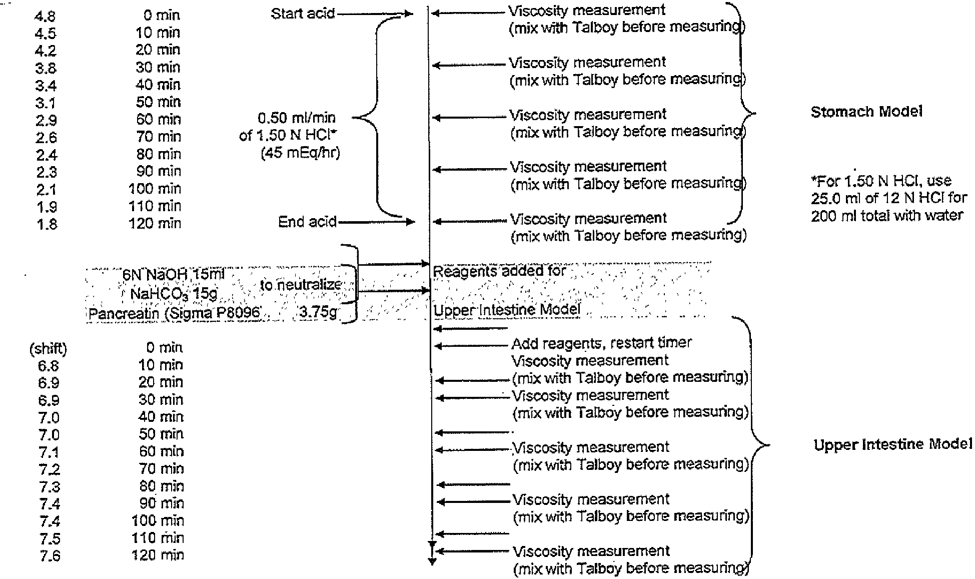

Viscosity measurement Unit Brookfield RVDV-II+Pro with temperature probe Mixing: Mix all samples with Talboy unit for 30 seconds on speed 2 before measuring viscosity

| | |
|---|---|
| Spindle Set: | RV |
| Spindle #: | 2-3 |
| Speed: | 50 |
| # Data Points: | 120⁻ |

*collect 125, disregard first 5

### Stomach/Intestinal Viscosity Sample Preparation Experimental Design

### Method (Neat Form):

1. Measure out stomach model ingredients (standardized to 1.25 liters) into stomach vessel and place in waterbath (37.5 °C)

| | |
|---|---|
| i. Water, DI | 277.5 ml |
| ii. 0.2M potassium hydrogen phthalate (40.844g potassium hydrogen phthalate in 1 liter water | 207.5 ml |
| iii. 1.0M NaOH (Sigma 930-65) | 18.8 ml |
| iv. Gastric mucosa mucin solution (1.5g gastric mucosa mucin in 50 ml water) | 43.1 ml |
| v. 0.2% pepsin solution (0.20g pepsin in 100 ml water) | 43.1 ml |

2. Heat 2 sets of 300g of DI water to 37 °C in waterbath
3. Weigh out required amount of Composite (see table below)
4. Divide the blended material into 2 equal portions
5. Add the 300g of pre-heated water from step 2 and half the active blend weighed out in step 4 to stomacher bag
6. Run stomacher for 120 seconds at speed 6 (with paddles set at 15.8mm)
7. Add contents of stomacher bag to stomach model solution prepared in step 1
8. Add the 2nd 300g of pre-heated water from step 2 and the other half of the blend to the same stomacher bag
9. Run stomacher for 120 seconds at speed 6 (with paddles set at 15.8mm)
10. Add contents of stomacher bag to the same stomach model solution prepared in step 1 (already containing ingredients from step 7)
11. Mix sample for 30 seconds using the Talboy stirrer on speed 2. The stirrer should be position in the middle of the vessel, with the mixing paddle 15cm below the rim of the vessel (lined up with mark on stirrer shaft). All sediment should be stirred up.
12. Measure sample viscosity (see below for test method)

### Composite (no blending with Maltodextrin)

| **Composite Composition** | **Dose Level (g/liter stomach)** | **Dose Level stomach) (g/1.25 liter** | **Notes** |
|---|---|---|---|
| 80:20 Starch:Hydrocolloid | 30 | 37.5 | Level standardized to 6g/liter stomach of hydrocolloid |
| 70:30 Starch:Hydrocolloid | 20 | 25 | Level standardized to 6g/liter stomach of hydrocolloid |

### Viscosity Settings:

| | |
|---|---|
| • | Unit: Brookfield RVDV-II+Pro with temperature probe |
| • | Mixing: Mix all samples with Talboy unit for 30 seconds on speed 2 before measuring viscosity |
| • | Spindle Set: RV |
| • | Spindle #: 2 or 3 |
| • | Speed: 50 |
| • | # Data Points: 120* |
| | *collect 125: disregard first 5 |

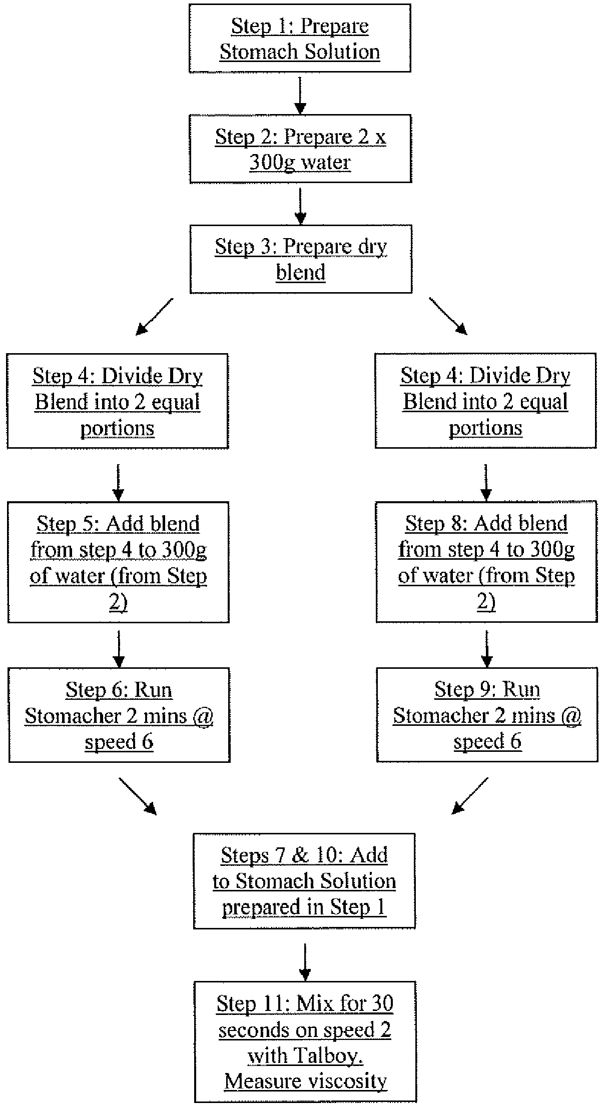

### EXAMPLE 1- Heat-Moisture Treated Whole Grain-Guar Gum Complexes:

A representative heat-moisture treatment was conducted under the following conditions. 80 parts of Hi-maize® Whole Grain Corn Flour was combined with 20 parts of Guar Gum HV in a standard bench top KitchenAid® mixer bowl. Ingredients were mixed on low speed with paddle attachments for 7 to 10 minutes to ensure uniformity. The mixture was sprayed in a uniform manner with 20 parts of ambient temperature distilled water. Mixing was continued for an additional 30 minutes. Moisture was checked to confirm 25% total moisture. Hydrated mixture was placed into a sealed aluminum dish and placed into a Despatch oven (Despatch Oven Co. Minneapolis, MN). Mixture was heated to 90 °C and held at 90 °C for 2 hours. After time elapsed, the final sample was cooled down and then air-dried to a final moisture content of 10% to 13%. The dried sample was ground using a coffee grinder; ground sample was screened using a US mesh 20 sieve. Material through US mesh 20 sieve was used for in vitro and applications screening.

The composition of Whole Grain-Guar Gum dry blend is set forth in Table 1 below.

**Table 1- Whole Grain-Guar Gum Complex Ingredients and Dry Blend Formulation**

| Ingredient | Level (%) | Manufacturer | Lot |
|---|---|---|---|
| Hi-maize® WGCF | 80.00 | NSFI | BHI 2000 |
| Coyote Brand™ Guar Gum HV | 20.00 | Gum Technology Corp. | 07F5A001Z |

### In vitro Glucose Release (GR) using a modified Englyst Assay

The results in Table 2 demonstrate that starch digestion was decreased as evidenced by the increase in RS content by heat-moisture treatment of Hi-maize® Whole Grain Corn Flour in the presence of guar gum. HMT WGCF-Guar Gum complex showed ≈ 10% and 26% higher RS content vs. dry blend control and native WGCF, respectively.

**Table 2- In vitro Glucose Release Results for Hi-maize® WGCF-Guar Gum Complexes**

| Ingredient | Moisture (%) | RS (% db) |
|---|---|---|
| WGCF (Native) | 11.5 | 60 |
| WGCF-Guar Dry blend | 11.5 | 73 |
| WGCF-Guar HMT Complex | 11.0 | 81 |

| | | |
|---|---|---|
| ¹ Based on total starch content | | |

Although RS content is an important performance criteria, gastrointestinal (GI) viscosifying ability and organoleptics are also important when considering commercial viability of such ingredients. For those determinations, test complexes and various controls were formulated into cold processed bars, and then tested for in vitro GI viscosity and for sensory attributes.

### Bar Formulations and Preparations

A cold form snack bar formulated with test ingredients and controls was used to evaluate textural attributes and measure in vitro stomach viscosity. The wet (ingredient) phase, consisting of corn syrup (63 DE), fig paste (23% moisture) and orange flavor (added post-heating), was prepared by heating to 60 °C (140 °F) to soften and aid blend uniformity. The heated wet phase was then added to the pre-blended dry phase consisting of rolled oats, granulated sugar, rice flour and salt. The blended mass was then portioned into 40g pieces and transferred into bar molds. The bars were formulated to deliver a soft texture, with a moisture content of ≈18% and a water activity of less than 0.60. Further refinement work was carried out on the snack bar formulation to develop a Control for stomach model and for sensory evaluations. A Control bar was formulated to match the nutritional profile, solids content and texture/firmness of the bars with complexes, and was evaluated as a reference point. To achieve this, the rice flour was decreased and the sugar and rolled oats levels increased to bring up the solids content and balance the carbohydrate level (target 80% carbohydrate). Whey protein (Instantized BiPRO® from Davisco Foods International, Inc.) was added at 0.09% to balance the protein level (target 5% protein) and canola oil was added at 1.10% to balance the fat level (target 2.5% fat).

Bar formulations are shown in Table 3 below.

**Table 3- Bar Formulations Used for Evaluation of HMT WGCF-Guar Complexes**

| **Ingredient** | **Control** | **Dry Blend or Complex** |
|---|---|---|
| Dries | | |
| Guar Gum* | --- | 4.616 |
| WGCF* | --- | 18.464 |
| Rolled Oats, Chopped | 21.600 | 16.014 |
| Sugar, EFG | 9.090 | 7.964 |
| Rice Flour (White) | 14.175 | --- |
| Whey Protein (BiPRO®) | 0.090 | --- |
| Salt | 0.045 | 0.043 |
| **Total** | **45.000** | **47.101** |

| Wets | | |
|---|---|---|
| Fig Paste (23% M) | 27.528 | 26.476 |
| Corn Syrup (DE 63) | 26.043 | 26.106 |
| Canola oil | 1.100 | --- |
| Nat. Orange Flv. WONF | 0.330 | 0.317 |
| **Total** | **55.001** | **52.899** |

| | | |
|---|---|---|
| ** In the complex, these two ingredients are in the form of a complex as prepared in Example 1* | | |

### Sensory Evaluation:

Sensory evaluation of snack bars was conducted by a sensory panel consisting of the same five individuals. Baseline sensory scores were established for the Control bar. Heat-moisture treated complexes and dry blend controls were evaluated in the Control bar base. The serving size of the bars was adjusted to accommodate for the different compositions of the materials, standardizing the total amount of control bar base used to 33.3g per serving. This resulted in a serving size of 43.3g for the 80:20 complexes (4.6% gum level by total formulation). This adjustment was made to keep the same amount of Control bar base being added into the Stomach/Intestine Viscosity Model for each complex run.

Four attributes, hardness, chewiness, gumminess and off-flavor were rated on a 9-point hedonic scale. For gumminess and off-flavor, a score of 4 was classified as borderline acceptable and 5 or above as unacceptable. Chewiness and hardness were ranked based on personal perception of bars, for example a granola bar being a 9 and a fruit bar being a 1. Snack bar samples were made on the bench top the day before evaluation (stored in a sealed container at room temperature) to allow the bars to equilibrate. Sensory scores were averaged across the five panelists to allow comparisons to be made. The key attributes identified as primary modes of failure were gumminess and off-flavor.

Sensory results in Table 4 demonstrate bars made with HMT Whole Grain-Guar gum complex scored lower with regard to gumminess and off-flavor vs. bars made with unprocessed dry blend.

**Table 4- Sensory Testing Results for Bars Made with Whole Grain-Guar Gum Complex vs. Dry Blend Control**

| Attribute | Control | Complex 14220:71-18 | Dry Blend 14220:71-13 |
|---|---|---|---|
| Gumminess | 2.3 | 3.0 | 4.8 |
| Off-flavor | 1.2 | 2.0 | 3.0 |

Table 5 shows bars made with HMT Whole Grain-Guar Gum complex exhibited ≈ 20% higher RS compared to bars prepared with dry blend, indicating RS content remains relatively intact in cold form bar application.

**Table 5- In vitro Glucose Release Results for Cold Form Bars Prepared with Whole Grain-Guar Gum Complex and Dry Blend Control**

| Ingredient Complex | Moisture (%) | RS (% wb) |
|---|---|---|
| Placebo Bar FN315039 | 12.5 | 32 |
| WGCF-Guar Gum Dry blend | 13.4 | 31 |
| WGCF-Guar Gum Complex | 12.9 | 39 |

### In Vitro Stomach/Intestinal Viscosity

Bars containing test ingredients were run through the stomach model assay as detailed above. All samples, including the fully hydrated guar gum gold standard reference, were assayed at equivalent guar gum levels. Intestinal phase viscosity data is presented as % area under the curve (AUC) vs. the fully hydrated guar gum reference. The data in Table 6 shows the gum from the complex displayed 100% of viscosifying power when compared to the fully hydrated gum reference. Guar gum is fully hydrated using a standard process known in the art.

**Table 6- HMT Whole Grain-Guar Gum Complex Stomach Viscosity Model % of Gold Standard Intestinal Area under the Curve in Bar Application**

| Ingredient Complex | % of Gold Standard with Bar Solids (Intestine) |
|---|---|
| Guar Gum | 100 |
| Gold Standard¹ | |
| WGCF-Guar gum | 100 |
| HMT Complex | |

| | |
|---|---|
| ¹ *Gold Standard is 6 g*/*L guar gum pre-hydrated and added to bar solids (7.5 grams of pre-hydrated guar gum* *+ 125 grams of bar base per 1.25L)* | |

### TDF Analysis:

TDF analysis of the neat ingredients was performed to determine if the HMT process would increase TDF content and to further characterize these materials. Table 7 shows TDF content for HMT Whole Grain-Guar Gum complex was ≈ 31% and 7% higher vs. native WGCF and dry blend, respectively.

**Table 7- TDF Results for HMT Whole Grain-Guar Gum Complex and Dry Blend by AOAC Method 991.43 (Medallion Labs)**

| Ingredient | TDF (% db) |
|---|---|
| Hi-maize® WGCF | 31 |
| WGCF-Guar Gum | 42 |
| Dry blend | |
| WGCF-Guar Gum | 45 |
| Complex | |

## Claims

1. A complex comprising a whole grain and a hydrocolloid.

2. The complex of claim 1, wherein the whole grain is a high amylose whole grain,

3. The complex of claim 1, wherein the whole grain is a high amylose corn whole grain.

4. The complex of any one of claims 1-3, wherein the hydrocolloid is a non-ionic gum.

5. The complex of claim 4, wherein the gum is selected from the group consisting of guar gum, konjac, locust bean gum, tara gum.

6. The complex of claim 4, wherein the gum is guar gum.

7. The complex of any one of claims 1-6 wherein the complex has a ratio (w/w) of whole grain:hydrocolloid of at least 90:10.

8. A process of making the complex of any one of claims 1-7 comprising: mixing the whole grain and hydrocolloid to form a blend; and heat-moisture treating the blend to form a complex.

9. A food product comprising the complex of any one of claims 1-7 and an additional edible ingredient.

10. The food product of claim 9, wherein the complex is in an amount of from 5 to 75% (w/w).

11. A method of increasing satiety comprising consuming at least 7.5 grams of the complex of any one of claims 1-7.

12. The method of claim 11, wherein caloric intake is decreased by at least 10% over the at least two hours following consumption compared to caloric intake following consumption of a readily digestible 10 DE maltodextrin of equal caloric content.

13. Use of the complexes of any one of claims 1-7 to increase satiety.
